(19) Europäisches Patentamt
European Patent Office
Office européen des brevets

(11) Veröffentlichungsnummer: **0 135 088**
**B1**

(12) # EUROPÄISCHE PATENTSCHRIFT

(45) Veröffentlichungstag der Patentschrift: **04.10.89**

(51) Int. Cl.⁴: **C 07 D 498/04**

(21) Anmeldenummer: **84109070.7**

(22) Anmeldetag: **01.08.84**

(54) **7-Amino-3(1-pyridiniomethyl)-8-oxo-5-oxa-1-azabicyclo(4.2.0)oct-2-en-2-carboxylate, Verfahren zu ihrer Herstellung und ihre Verwendung als Zwischenprodukte zur Synthese von beta-Lactam-antibiotika.**

(30) Priorität: **13.08.83 DE 3329427**

(43) Veröffentlichungstag der Anmeldung: **27.03.85 Patentblatt 85/13**

(45) Bekanntmachung des Hinweises auf die Patenterteilung: **04.10.89 Patentblatt 89/40**

(84) Benannte Vertragsstaaten: **AT BE CH DE FR GB IT LI NL SE**

(56) Entgegenhaltungen:
**EP-A-0 049 448**
**DE-A-3 137 038**
**US-A-4 325 951**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Hartwig, Wolfgang, Dr., Pahlkestrasse 3, D-5600 Wuppertal 1 (DE)**
Erfinder: **Häbich, Dieter, Dr., Krummacherstrasse 82, D-5600 Wuppertal (DE)**

**EP 0 135 088 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die vorliegende Erfindung betrifft neue 7-Amino-3[1-pyridiniomethyl]8-oxo-5-oxa-1-azabicyclo[4.2.0]oct-2-en-carboxylate, ein Verfahren zu ihrer Herstellung und ihre Verwendung als Zwischenprodukte insbesondere zur Synthese von β-Lactamantibiotika.

Die durch die Erfindung zur Verfügung gestellten (6R)-7β-Amino-3[1-pyridiniomethyl]-8-oxo-5-oxa-1-azabicyclo[4.2.0]oct-2-en-2-carboxylate haben die allgemeine Formel (1)

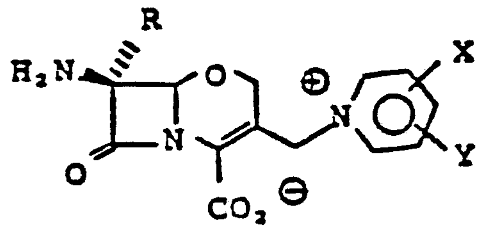

(1)

in welcher

R für Wasserstoff oder Methoxy und

X und Y unabhängig voneinander für folgende Bedeutungen stehen:

Wasserstoff; Halogen; Cyano; Hydroxy; $C_1$-$C_4$-Alkoxy; Phenoxy; Amino, $C_1$-$C_4$-Alkylamino, $C_1$-$C_4$-Dialkylamino oder $C_1$-$C_4$-Alkanoylamino; Carboxy; $C_1$-$C_4$-Alkanoyl; Benzoyl; Phenyl-$C_1$-$C_4$-alkanoyl; $C_1$-$C_4$-Alkoxycarbonyl; Sulfamoyl; Carbamoyl; N-$C_1$-$C_4$-alkyl-, N,N-$C_1$-$C_4$-dialkyl- oder N-$C_1$-$C_4$-alkoxycarbonylcarbamoyl; Allophanoyl und 4-$C_1$-$C_4$-Alkylallophanoyl; $C_1$-$C_4$-Alkylsulfonyl; $C_1$-$C_4$-Alkylthio; gegebenenfalls 1- bis mehrfach durch Hydroxy, $C_1$-$C_4$-Alkoxy, Halogen, Cyano, Carboxy und $C_1$-$C_4$-Alkoxycarbonyl, $C_1$-$C_4$-Alkanoyl, $C_1$-$C_4$-Alkylsulfonyl oder -sulfinyl, Amino, oder mono- oder di-Alkyl-$C_1$-$C_4$-amino, $C_1$-$C_4$-Alkoxyimino substituiertes $C_1$-$C_6$-Alkyl oder Alkenyl, $C_3$-$C_7$-Cycloalkyl oder -Cycloalkenyl, Phenyl, Heterocyclyl mit 5 - 6 Ringen und 1 bis 4 Heteroatomen aus der Gruppe O, N und/oder S, Phenylalkyl oder Heterocyclylalkyl in der vorgenannten Bedeutung mit 1 bis 2 C-Atomen im Alkylteil, wobei die Reste Wasserstoff/Wasserstoff, Amino/Wasserstoff und Carbamoyl/Wasserstoff ausgenommen sind,

X und Y zusammen stehen für einen am Pyridinring in 2,3 bzw. 4,5 Stellung anellierten carbocyclischen Rest mit 5 - 7 Kohlenstoffatomen, die gegebenenfalls durch Heteroatome O, S oder N ersetzt sind.

Die genannten Alkyl- und Alkenylreste können unverzweigt oder verzweigt sein. Sie haben besonders bevorzugt 1 - 4 C-Atome. Die Cycloalkyl- und Cycloalkenylreste haben besonders bevorzugt 5 oder 6 C-Atome.

Unter Alkenyl und Cycloalkenyl werden erfindungsgemäß auch mehrfach ungesättigte Reste, besonders aber ein- und zweifach ungesättigte Reste verstanden.

Heterocyclyl steht insbesondere für heterocyclische 5- und 6-Ringe, die vorzugsweise 1 oder 2 Heteroatome aus der Gruppe O, N und/oder S enthalten. Sie können gesättigt oder ein- oder mehrfach ungesättigt sein. In der Bedeutung Phenylalkyl oder Heterocyclylalkyl hat der Alkylteil insbesondere 1 - 2 C-Atome. Heterocyclyl hat die zuvor genannte Bedeutung.

Die Reste Alkyl, Alkenyl, Cycloalkyl, Cycloalkenyl, Aryl, Heterocyclyl und Aralkyl, letzterer sowohl im Arylwie im Alkylteil, sowie der annellierte Ring können vorzugsweise 1- bis 2-fach, insbesondere einfach substituiert sein.

Halogen in der Bedeutung von X und Y und als Substituent für Alkyl, Alkenyl, Cycloalkyl und -alkenyl, Aryl, Heterocyclyl und Aralkyl und den annellierten Ring steht vorzugsweise für Fluor, Chlor oder Brom.

Beispiele für die Bedeutungen X und Y sind:

4-Methyl, 3-Methyl, 2-Methyl, 4-Ethyl, 3-Ethyl, 2-Ethyl, 4-Isopropyl, 3-Isopropyl, 2-Isopropyl, 4-[1-Ethyl]-propyl, 3-[1-Ethyl]propyl, 2-[1-Ethyl]propyl, 4-Prop-2-enyl, 3-Prop-2-enyl, 3-[2-Methylprop-2-enyl], 4-[2-Methyl]prop-2-enyl, 3-Prop-1-enyl, 4-But-3-enyl, 4-Cyclohex-1-enyl, 3-Cyclohex-1-enyl, 4-Cyclopent-1-enyl, 4-Cyclohex-1-enyl, 4-Cyclopent-1-enyl, 3-Cyclopent-1-enyl, 4-Cyclohept-1-enyl, 3-Cyclohept-1-enyl, 4-[4-Methyl]cyclohex-1-enyl, 3-[4-Methyl]cyclohex-1-enyl, 4-Cyclopropyl, 3-Cyclopropyl, 2-Cyclopropyl, 4-Cyclopentyl, 3-Cyclopentyl, 2-Cyclopentyl, 4-Cyclohexyl-, 3-Cyclohexyl, 2-Cyclohexyl, 4-[2-Hydroxy]propyl, 4-[3-Hydroxy]-propyl, 4-[1-Hydroxy]ethyl, 3-[1-Hydroxy-1-methyl]ethyl, 4-[1-Hydroxy-1-methyl]ethyl, 3-[3-Hydroxy]propyl, 3-[1-Hydroxy]cyclohexyl, 3-[1-Hydroxy]cyclopentyl, 4-[1-Hydroxy]cyclohexyl, 4-[1-Hydroxy]cyclopentyl, 3-[1-Hydroxy]cycloheptyl, 3-[1-Hydroxy-4-methyl]cyclohexyl, 4-[1-Hydroxy-cyclopentyl]methyl, 4-[1-Hydroxycyclohexyl]methyl, 2-[Dihydroxymethyl]methyl, 4-Methoxy, 3-Methoxy, 2-Methoxy, 4-Ethoxy, 3-Ethoxy, 2-Ethoxy, 3-Isopropyloxy, 4-Isopropyloxy, 3-Propyloxy, 4-Propyloxy, 2-Propyloxy, 4-[2-Ethoxy]ethyl, 4-[Methoxy]methyl, 2-[2-Ethoxy]ethyl, 4-[2,5-Dioxy]cyclopentyl, 3-[2,5-Dioxy]cyclopentyl, 3-[2,5-Dioxy-1-methyl]cyclopentyl, 4-[2,5-Dioxy-1-methyl]cyclopentyl, 2-Methylthio, 3-Methylthio, 4-Methylthio, 4-[Methylthio]methyl, 3-Methylsulfinylmethyl, 4-Methylsulfinylmethyl, 3-Methylsulfonylmethyl, 3-Hydroxy, 4-Hydroxy, 2-Hydroxy, 3-Acetyl, 4-Acetyl, 3-Benzoyl, 4-Benzoyl, 4-Acetamidoyl, 4-[1,γ-Pyridonyl], 4-[2-Pyridyl], 3-Ethoxycarbonyl, 4-Ethoxycarbonyl, 3-Carboxymethyl, 3-[Ethoxycarbonyl]methyl, 4-[4-Ethyl]allophanoyl, 4-Carbamoylmethyl, 3-Carbamoyl, 4-Carbamoyl, 3-[N-Ethoxycarbonyl]carbamoyl, 4-[N-Ethoxycarbonyl]carbamoyl, 4-[N-Ethyl]carbamoyl, 3-[N,N-Diethylcarbamoyl], 4-[N,N-Diethylcarbamoyl], 4-Cyano, 4-Carboxy-, 4-Sulfamoyl, 4-[2-Kaliumsulfonato]ethyl, 4-[1-Acetonyl], 4-Trifluormethyl, 3-Trifluormethyl, 4-[3-Chlor]propyl, 3-[3-Chlor]propyl, 3-Cyanomethyl, 4-[1-Sulfo-1-hydroxy]methyl, 4-[E-2-Ethoxycarbonyl]ethenyl, 3-[E-2-Methoxycarbonyl]ethenyl, 4-[2-Aza-3-oxa]hex-1-enyl, 4-[2-Aza-3-oxa-4-phenyl]hex-1-enyl, 3-Chlor, 4-Chlor, 2-Chlor, 4-Brom, 3-Brom, 2-Brom, 4-Fluor, 3-Fluor, 2-Fluor.

Besonders bevorzugt stehen X und Y für Wasserstoff. Ferner sind erfindungsgemäß solche Verbindungen (1) bevorzugt, in denen X = H ist und eine der vorstehenden Bedeutungen hat.

Im Falle von Disubstitution am Pyridinring ($X = Y \neq H$) sind besonders bevorzugt die Verbindungen, in denen X 4-Methyl und Y 2-Methyl oder 3-Methyl; X 4-Hydroxyl und Y 2-Methyl; X 4-Hydroxy und Y 4-Methyl; X 3-Methyl und Y 4-Methoxy oder X 4-Methyl und Y 3-Methoxy bedeutet.

Die erfindungsgemäßen Verbindungen der allgemeinen Formel (1) und jene, worin X und Y die Reste Wasserstoff/Wasserstoff, Amino/Wasserstoff und Carbamoyl/Wasserstoff darstellen, können dadurch erhalten werden, daß man in

(6R)-7β-Acylamino-3[1-pyridiniomethyl]-8-oxo-5-oxa-1-azabicyclo[4.2.0]oct-2-en-2-carboxylaten der allgemeinen Formel (2),

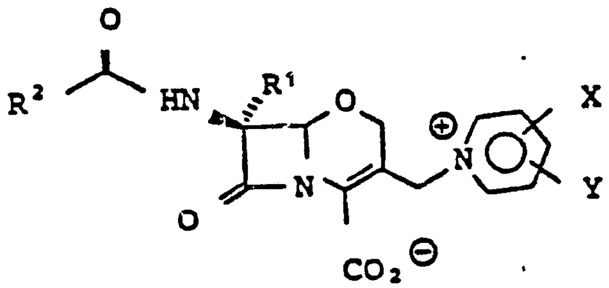

(2)

in welcher

$R^1$, X und Y die oben angegebene Bedeutung haben und

O
‖
$R^2$-C- für einen Acylrest steht,

den Acylrest in an sich in der β-Lactamchemie bekannten Weise, vorzugsweise enzymatisch, abspaltet.

$R^2$ bedeutet bevorzugt gegebenenfalls substituiertes Phenyl. Ganz besonders bevorzugt steht $R^2$ für Phenyl, Phenylmethyl oder Phenoxymethyl.

Die Verbindungen der allgemeinen Formel (2) können dadurch erhalten werden, daß man Verbindungen der allgemeinen Formel (3),

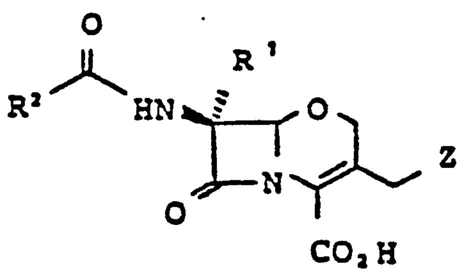

(3)

in der

Z für einen nucleofuge Abgangsgruppe, vorzugsweise für Chlor steht und $R^1$ bzw. $R^2$ die oben angegebene Bedeutung haben,

mit Pyridin bzw. einem in der Formel (1) angegebenen und mit X bzw. Y näher bezeichneten substituierten Pyridin in einem geeigneten inerten organischen Lösungsmittel, vorzugsweise Tetrahydrofuran umsetzt.

Die Verbindungen der allgemeinen Formel (3) können dadurch erhalten werden, daß man in den Verbindungen der allgemeinen Formel (4)

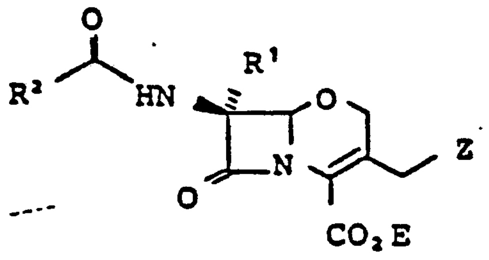

(4)

in der

$R^1$, $R^2$ und Z die oben näher bezeichnete Bedeutung haben und E für eine in der β-Lactamchemie üblicherweise verwendete Säureschutzgruppe, vorzugsweise für Diphenylmethyl oder tert.-Butyl steht,

die Säureschutzgruppe in Analogie zu bekannten Verfahren abspaltet.

Vorzugsweise geschieht dies im Falle des Benzhydrylesters bzw. tert.-Butylesters durch Behandeln mit Trifluoressigsäure in einem inerten organischen Lösungsmittel.

Die Verbindungen der allgemeinen Formel (4) mit X = Cl und OAc sind bereits bekannt [vergl. T. Aoki et. al., Heterocycles 15, (1981) 409; Deutsche Offenlegungsschrift 2 806 457 und US Patentschrift 4 342 685].

Die Verbindungen der allgemeinen Formel (4) mit X = Chloracetyl und Dichloracetyl sind noch nicht bekannt, können aber analog den in der oben angeführten Literatur beschriebenen Verfahren synthetisiert werden, indem man den im Verlauf der Synthesesequenz auftretenden 3-Hydroxymethylcephamcarbonsäureester mit Chloracetylchlorid oder Dichloracetylchlorid anstelle von Acetylchlorid umsetzt.

Verwendet man (6R,7S)-7-Phenylacetylamino-3-chlormethyl-5-oxa-8-oxo-1-azabicyclo[4.2.0]oct-2-en-2-carbonsäurediphenylmethylester (4a) und Pyridin als Ausgangsstoffe, so kann der Reaktionsablauf durch das folgende Formelschema wiedergegeben werden.

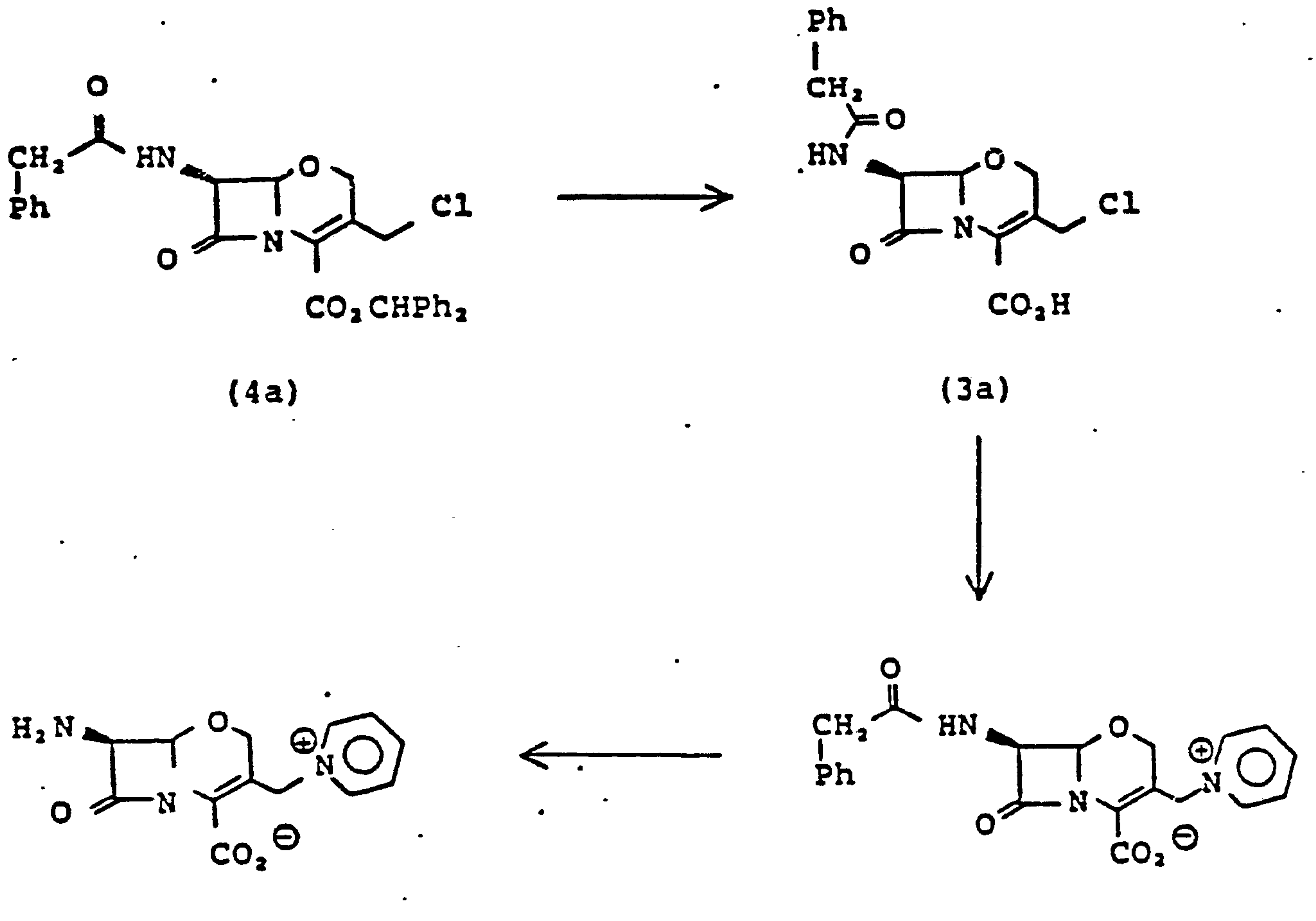

(4a) (3a)

(1a) (2a)

Für die nucleofuge Abgangsgruppe Z steht Chlor, Brom, Jod oder ein Chloracetyl-, Dichloracetyl-, Trichloracetyl-, O-Mesy-, O-Tosyl-, O-Trifluormesyl- oder ein (Methoxycarbonyl)acetyloxyrest.

Bei der Umsetzung von Verbindungen der Formel (4) zu Verbindungen der Formel (3) kommen alle Reagenzien in Betracht die die in der β-Lactamchemie üblicherweise verwendeten Säureschutzgruppen selektiv zu spalten vermögen.

Vorzugsweise verwendet man im Falle des Benzhydrylesters oder tert.-Butylesters wasserfreie Trifluoressigsäure in Gegenwart von Anisol.

Als Verdünnungsmittel kommen alle inerten organischen Lösungsmittel in Frage, wie z. B. halogenierte Kohlenwasserstoffe wie Dichlormethan, Trichlormethan, Tetrachlormethan oder 1,2-Dichlorethan.

Die Umsetzungen erfolgen im allgemeinen bei Temperaturen zwischen -70°C und 40°C vorzugsweise zwischen -10°C und 0°C.

Bei der Umsetzung der Verbindungen der Formel (3) zu den Verbindungen der Formel (2) mit Pyridin bzw. einem in der Formel (2) bzw. (1) angegebenen und mit X bzw. Y näher bezeichneten substituierten Pyridin kommen alle inerten organischen Verdünnungsmittel als Lösungsmittel in Frage. Hierzu gehören halogenierte Kohlenwasserstoffe wie Dichlormethan, Trichlormethan oder 1,2-Dichlorethan, cyclische Ether wie Tetrahydrofuran und Dioxan oder dipolar aprotische Lösungsmittel wie z. B. Dimethylformamid oder Mischungen derselben.

Die Umsetzungen erfolgen im allgemeinen bei Temperaturen zwischen -15°C und 40°C, vorzugsweise bei 15°C - 25°C.

Bei der Umsetzung der Verbindungen der Formel (2) zu den erfindungsgemäßen Verbindungen der Formel (1 ) kommen alle in der β-Lactamchemie üblichen chemischen oder enzymatischen Verfahren zur Abspaltung eines N-Acylrestes in Frage.

Die chemische Abspaltung des N-Acylrestes in den Verbindungen der Formel (2) führt man vorzugsweise so durch, daß man die Verbindung (2) zunächst mit einem Silylierungsmittel wie Chlor- oder Jod-trimethylsilan, N-Trimethylsilylacetamid, N-Methyl-N-trimethylsilylacetamid oder N-Methyl-N-trimethylsilyltrifluoracetamid in den entsprechenden Silylester überführt.

Anschließend wird - ohne den Silylester zu isolieren - analog zu bekannten Verfahren der N-Acylrest abgespalten. Hierzu setzt man den Silylester mit einem anorganischen oder organischen Säurechlorid, vorzugsweise Phosphorpentachlorid, Phosphoroxychlorid, Thionylchlorid, Oxalylchlorid oder Phosgen um und gibt anschließend einen niederen aliphatischen Alkohol wie Methanol, Ethanol oder Isobutanol oder ein Diol wie z. B. 1,3-Butandiol zu.

Als Verdünnungsmittel bei der obengenannten Umsetzung kommen alle inerten organischen Lösungsmittel in Frage, vorzugsweise halogenierte Kohlenwasserstoffe wie z. B. Dichlormethan oder Dichlorethan oder cyclische Ether wie z. B. Tetrahydrofuran und Dioxan oder Ethylacetat oder Mischungen derselben.

Die Umsetzungen erfolgen im allgemeinen bei Temperaturen zwischen -70°C und 40°C, vorzugsweise bei -30°C und 35°C.

Die enzymatische Abspaltung des N-Acylrestes in den Verbindungen der Formel (2) führt man vorzugsweise so durch, daß man die Verbindungen der Formel (2) in Wasser bei pH 6 bis 8 vorzugsweise bei pH = 7.8 mit Penicillin G- oder V-Acylase in Analogie zu bekannten Verfahren behandelt.

Zur Aufrechterhaltung des pH-Wertes können alle anorganischen Basen herangezogen werden, vorzugsweise Natriumhydroxyd, Lithiumhydroxyd, Kaliumhydroxyd oder Cäsiumhydroxyd.

Ebenfalls in Betracht kommen organische Basen wie tert.-Ammoniumbasen wie vorzugsweise Triethylamin, tri-n-Butylamin oder N-Ethyldiisopropylamin.

Die Umsetzung wird bei Temperaturen zwischen 10°C und 30°C, vorzugsweise bei 25°C durchgeführt.

Die erfindungsgemäßen Verbindungen der allgemeinen Formel (1) können als Zwischenprodukte zur Synthese von β-Lactamantibiotika herangezogen werden. Hierzu wird in bekannter Weise die 7β-Aminogruppe mit organischen Carbonsäuren oder deren aktiven Derivaten acyliert um Verbindungen mit anderen Seitenketten als der Acylgruppe $R^2$-CO zu erhalten.

## Beispiele

### Beispiel 1

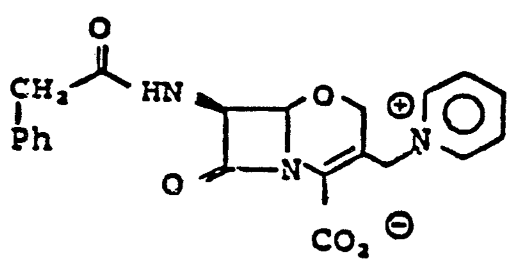

(6R,7S)-7-Phenylacetylamino-3[1-pyridiniomethyl]-8-oxo-5-oxa-1-azabicyclo[4.2.0]oct-2-en-2-carboxylat

Zu einer auf -10°C gekühlten Lösung von 120 g (2,3 mmol) (6R,7S)-7-Phenylacetylamino-3[chloromethyl]-8-oxo-5-oxa-1-azabicyclo[4.2.0]oct-2-en-2-carbonsäurediphenylmethylester in 14 ml absoluten Dichlormethan und 5 ml abs. Anisol tropfte man unter Stickstoffatmosphäre 7 ml wasserfreie Trifluoressigsäure so zu, daß die Temperatur -10°C nicht überstieg. Man rührte 30 min. bei -10°C, gab anschließend 10 ml abs. Benzol zu und engte im Vakuum (Badtemperatur 15°) auf ein Drittel des Volumens ein. Man verdünnte mit 10 ml absolutem Tetrahydrofuran, kühlte auf -15°C und gab 10 ml absolutes Pyridin zu. Man ließ auf Raumtemperatur kommen und rührte 20 h nach. Man gab 20 ml Tetrahydrofuran zu und ließ langsam und unter Rühren 150 ml Diethylether zulaufen. Der Niederschlag wurde unter Stickstoffatmosphäre abgesaugt, mit Ether gewaschen und 30 min. in 200 ml bidestilliertem Wasser ausgerührt. Es wurde vom Ungelösten abfiltriert und das Filtrat durch Zugabe von schwach basischen Ionenaustauscherharz MP 62 auf pH 6.4 gestellt. Nach Filtration und Gefriertrocknung der wässrigen Phase erhielt man 400 mg (43.8 %) der Titelbindung als hell-beiges Lyophilisat.

Rf ($CH_3CN/H_2O$ : 2/1) = 0.37

IR (KBr): $\gamma$ = 3427 - 3057 (br), 1778, 1666, 1620 $cm^{-1}$

1H-NMR (250 MHz, $[D_6]$-DMSO: $\delta$ = 3.46 (s; 2H, $CH_2Ph$); 4.12 und 4.41 (AB-System, $J_{AB}$ = 17.5 Hz; 2H, $OCH_2$); 5.09 (d, J = 4 Hz; 1H, 6-H); 5.40 (dd, J = 4 Hz, J = 10 Hz; 1H, 7-H); 5.19 und 5.95 (AB-System, $J_{AB}$ = 12.5 Hz; 2H, $CH_2N^{\ominus}$); 7.18 - 7.31 (m; 5H, Aromaten-H); 8.09 - 8.21 (m; 2H, 3.5-Pyridin-H); 8.54 - 8.64 (m; 1H, 4-Pyrid-H); 9.49 (d, J = 6 Hz; 2H, 2.6-Pyrid-H)

**Beispiel 2**

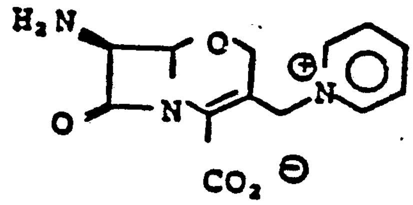

(6R,7S)-7-Amino-3[1-pyridiniomethyl]-8-oxo-5-oxa-1-azabicyclo[4.2.0]oct-2-en-2-carboxylat

400 mg (0.1 mmol) (6R,7S)-7-Phenylacetylamino-3[1-pyridiniomethyl]-8-oxo-5-oxa-1-azabicyclo[4.2.0]oct-2-en-2-carboxylat wurden in 20 ml Wasser gelöst, mit 400 mg Penicillin G-Acylase versetzt und bei Raumtemperatur am Autotitrator bei pH = 7.8 2 h gerührt. Man filtrierte ab, säuerte mit 0.2 N HCl auf pH = 2.5 an und extrahierte die wäßrige Phase dreimal mit je 20 ml Ethylacetat. Nach Gefriertrocknung der wäßrigen Phase erhielt man 224 mg (71 %) des Hydrochlorids der Titelverbindung als hellgelbes Lyophilisat.

Rf: ($CH_3CN/H_2O$ = 2/1) = 0.11

IR (KBr) $\gamma$ = 3451, 1770, 1632 $cm^{-1}$

$^1$H-NMR (250 MHz, $D_2O$): $\delta$ = 4.32 und 4.44 (AB-System, $J_{AB}$ = 15 Hz; 2H, $OCH_2$); 4.55 (d, J = 4 Hz; 1H, 6-H) 5.14 (d, J = 4 Hz; 1H, 7-H); 5.15 und 5.67 (AB-System, $J_{AB}$ = 15 Hz; 2H, $CH_2$-N+); 8.00 - 8.06 (m; 2H, 3.5-Pyrid.-H); 8.48 - 8.54 (m; 1H, 4-Pyrid.-H); 8.91 (d, J = 6 Hz; 2H, 2.6-Pyrid.-H)

**Beispiel 3**

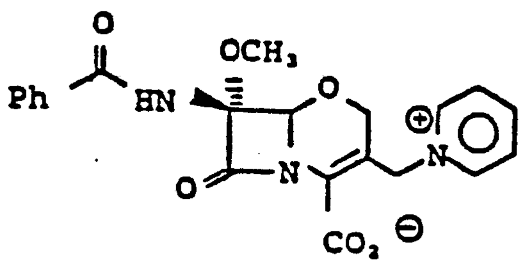

(6R,7S)-7-Benzoylamino-7-methoxy-3-[1-pyridiniomethyl]-8-oxo-5-oxa-1-azabicyclo[4.2.0]oct-2-en-2-carboxylat

Eingesetzt wurden 3.73 g (7 mmol) (6R,7S)-7-Benzoylamino-3-[chloromethyl]-8-oxo-5-oxa-1-azabicyclo-[4.2.0]oct-2-en-2-carbonsäurebenzhydrylester. Man verfuhr analog der Vorschrift für Beispiel 1 und erhielt 898 mg (31 %) der Titelverbindung als helles Lyophilisat.

Rf: ($CH_3CN/H_2O$ = 2/1) = 0.36

IR (KBr): 1770, 1660, 1615, 1514 $cm^{-1}$

$^1$H-NMR (250 MHz, $[D_6]$-DMSO): $\delta$ = 3.42 (s; 3H, $OCH_3$); 4.09, 4.38 (AB-System, J = 16 Hz; 2H, $CH_2O$); 5.08 (s; 1H; 6-H); 5.15 und 5.84 (AB-System, J = 15 Hz; 2H, $CH_2N^{\oplus}$); 7.4 - 7.6 (m; 3H, Phenyl), 7.9 (m; 2H, O-Benzoyl-H); 8.15 (m; 2H, 3.5-Pyrid.-H); 8.58 (m; 1H, 4-Pyrid.-H); 9.30 (s; 1H, NH); 9.46 (d, j = 6 Hz; 2H, 2.6-Pyrid.-H)

**Beispiel 4**

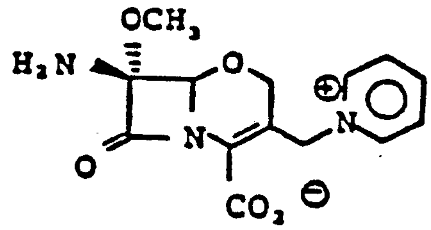

(6R,7S)-7-Amino-7-methoxy-3[1-pyridiniomethyl]-8-oxo-5-oxa-1-azabicyclo[4.2.0]oct-2-en-2-carboxylat

Zu einer Suspension von 835 mg (2 mmol) (6R,7S)-7-Benzoylamino-7-3[1-pyridiniomethyl]-8-oxo-5-oxa-1-azabicyclo[4.2.0]oct-2-en-2-carboxylat in 6 ml wasserfreiem Dichlormethan wurden nacheinander, 1.01 ml (8 mol) Dimethylanilin und 0.51 ml (4 mmol) Trimethylchlorsilan gegeben. Die Mischung wurde 1 h auf 35°C erwärmt und dann auf -30°C abgekühlt. Man gab 833 mg (4 mmol) $PCl_5$ zu und rührte 20 min. bei -30°C, 50 min. bei 0°C und 1 h bei 10°C. Anschließend kühlte man auf -30°C, gab 1.614 ml abs. 1.3 Butandiol zu und ließ im Kühlbad langsam auf 0°C kommen. Man verdünnte mit 200 ml Ether, saugte ab, wusch mit Ether nach und trocknete den Niederschlag am Hochvakuum über $P_4O_{10}$. Man erhielt das Hydrochlorid der Titelverbindung als helles Pulver.

IR (KBr): 1770 $cm^{-1}$

$^1H$-NMR (250 MHz, $D_2O$): $\delta$ = 3.4 (s, 3H, $OCH_3$); 4.22 und 4.34 (AB-System, $J_{AB}$ = 15 Hz, 2H, $OCH_2$); 4.9 (s; 1H, 6-H); 5.2 und 5.7 (AB-System, $J_{AB}$ = 15 Hz; 2H, $CH_2N^{\oplus}$), 8.50 - 8.51 (m; 1H, 4-Pyrid.-H); 8.01 - 8.07 (m; 2H, 3.5-Pyrid.-H), 8.90 (d, J = 6 Hz; 2H, 2.6-Pyrid.-H)

**Beispiel 5**

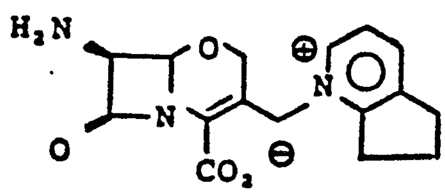

(6R,7S)-7-Amino-3-[(2,3-cyclopenteno-1-pyridinio)methyl]-8-oxo-6-oxa-1-azabicyclo[4.2.0]oct-2-en-2-carboxylat

Man verfuhr analog wie unter Beispiel 1 bzw. 2 beschrieben, nur setzte man statt Pyridin 2,3-Cyclopentenopyridin ein.

Ausgehend von 1.14 g (2.2 mmol) (6R,7S)-7-Phenylacetylamino-3-chloromethyl-8-oxo-5-oxa-1-azabicyclo[4.2.0]oct-2-en-2-carbonsäurebenzhydrylester erhielt man über zwei Stufen 0.72 g der Titelverbindung als hell-beiges Lyophilisat.

Rf: ($CH_3CN/H_2O$ = 2/1) = 0.12

IR (KBr): $\nu$ = 3450, 1770, 1631 $cm^{-1}$

$^1H$-NMR (200 MHz, $D_2O$): $\delta$ = 2.19 (hept. erkennbar als t, J = 7.5 Hz; 2H, $\beta$-Cyclopent.-H); 3.05 (t, J = 7. 5 Hz; 2H, $\gamma$-Cyclopent.-H); 3.21 (t, J = 7.5 Hz; 2H, $\alpha$-Cyclopent.-H); 4.44 (s, br.; 2H, $OCH_2$); 4.91 (d, J = 4 Hz; 1H, 6-H); 5.31 (d; J = 4 Hz, 1H, 7-H); 5.35 u. 5.75 (AB-System, $J_{AB}$ = 15 Hz; 2H, $CH_2$-$N^{\oplus}$); 7.63 (t, J = 7.5 Hz; 1H, 4-Pyrid.-H); 8.15 (d, J = 7.5 Hz; 1H, 5-Pyrid.-H), 8.44 J = 7.5 Hz; 1H, 6-Pyrid.-H)

**Patentansprüche**

1. Verbindungen der allgemeinen Formel (1)

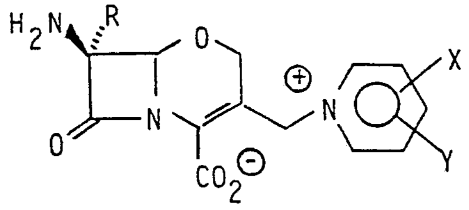

(1) .

in welcher

R für Wasserstoff oder Methoxy und

X und Y unabhängig voneinander für folgende Bedeutungen stehen:

Wasserstoff; Halogen; Cyano; Hydroxy; $C_1$-$C_4$-Alkoxy; Phenoxy; Amino, $C_1$-$C_4$-Alkylamino, $C_1$-$C_4$-Dialkylamino oder $C_1$-$C_4$-Alkanoylamino; Carboxy; $C_1$-$C_4$-Alkanoyl; Benzoyl; Phenyl-$C_1$-$C_4$-alkanoyl; $C_1$-$C_4$-Alkoxycarbonyl; Sulfamoyl; Carbamoyl; N-$C_1$-$C_4$-alkyl-, N,N-$C_1$-$C_4$-dialkyl- oder N-$C_1$-$C_4$-alkoxycarbonylcarbamoyl; Allophanoyl und 4-$C_1$-$C_4$-Alkylallophanoyl; $C_1$-$C_4$-Alkylsulfonyl; $C_1$-$C_4$-Alkylthio; gegebenenfalls 1- bis mehrfach durch Hydroxy, $C_1$-$C_4$-Alkoxy, Halogen, Cyano, Carboxy und $C_1$-$C_4$-Alkoxycarbonyl, $C_1$-$C_4$-Alkanoyl, $C_1$-$C_4$-Alkylsulfonyl oder -sulfinyl, Amino, oder mono- oder di-$C_1$-$C_4$-Alkylamino, $C_1$-$C_4$-Alkoxyimino substituiertes $C_1$-$C_6$-Alkyl oder Alkenyl, $C_3$-$C_7$-Cycloalkyl oder -Cycloalkenyl, Phenyl, Heterocyclyl mit 5- bis 6-gliedrigen Ringen und 1 bis 4 Heteroatomen aus der Gruppe O, N und/oder S, Phenylalkyl oder Heterocyclylalkyl in der vorgenannten Bedeutung mit 1 bis 2 C-Atomen im Alkylteil, wobei die Reste Wasserstoff/Wasserstoff, Amino/Wasserstoff und Carbamoyl/Wasserstoff ausgenommen sind;

X und Y zusammen stehen für einen am Pyridinring in 2,3 bzw. 4,5 Stellung anellierten carbocyclischen Rest mit 5 - 7 Kohlenstoffatomen, die gegebenenfalls durch Heteroatome O, S oder N ersetzt sind.

2. Verbindungen nach Anspruch 1, dadurch gekennzeichnet, daß X für 4-Methyl und Y für 2-Methyl oder 3-Methyl steht.

3. Verbindungen nach Anspruch 1, dadurch gekennzeichnet, daß X für 4-Hydroxyl und Y für 2-Methyl oder X für 4-Hydroxy und Y für 4-Methyl oder X für 3-Methyl und Y für 4-Methoxy oder X für 4-Methyl und Y für 3-Methoxy steht.

4. Verfahren zur Herstellung von Verbindungen der allgemeinen Formel (1)

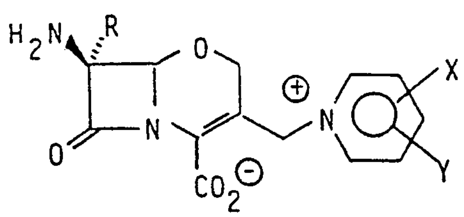

(1)

in welcher

R für Wasserstoff oder Methoxy und

X und Y unabhängig voneinander für folgende Bedeutungen stehen:

Wasserstoff; Halogen; Cyano; Hydroxy; $C_1$-$C_4$-Alkoxy; Phenoxy; Amino, $C_1$-$C_4$-Alkylamino, $C_1$-$C_4$-Dialkylamino oder $C_1$-$C_4$-Alkanoylamino; Carboxy; $C_1$-$C_4$-Alkanoyl; Benzoyl; Phenyl-$C_1$-$C_4$-alkanoyl; $C_1$-$C_4$-Alkoxycarbonyl; Sulfamoyl; Carbamoyl; N-$C_1$-$C_4$-alkyl-, N,N-$C_1$-$C_4$-dialkyl- oder N-$C_1$-$C_4$-alkoxycarbonylcarbamoyl; Allophanoyl und 4-$C_1$-$C_4$-Alkylallophanoyl; $C_1$-$C_4$-Alkylsulfonyl; $C_1$-$C_4$-Alkylthio; gegebenenfalls 1- bis mehrfach durch Hydroxy, $C_1$-$C_4$-Alkoxy, Halogen, Cyano, Carboxy und $C_1$-$C_4$-Alkoxycarbonyl, $C_1$-$C_4$-Alkanoyl, $C_1$-$C_4$-Alkylsulfonyl oder -sulfinyl, Amino, oder mono- oder di-$C_1$-$C_4$-Alkylamino, $C_1$-$C_4$-Alkoxyimino substituiertes oder $C_1$-$C_6$-Alkyl oder Alkenyl, $C_3$-$C_7$-Cycloalkyl oder -Cycloalkenyl, Phenyl, Heterocyclyl mit 5- bis 6-gliedrigen Ringen und 1 bis 4 Heteroatomen aus der Gruppe O, N und/oder S, Phenylalkyl oder Heterocyclylalkyl in der vorgenannten Bedeutung mit 1 bis 2 C-Atomen im Alkylteil;

X und Y zusammen stehen für einen am Pyridinring in 2,3 bzw. 4,5 Stellung anellierten carbocyclischen Rest mit 5 - 7 Kohlenstoffatomen, die gegebenenfalls durch Heteroatome O, S oder N ersetzt sind,

dadurch gekennzeichnet, daß man

a) in den Verbindungen der allgemeinen Formel (4)

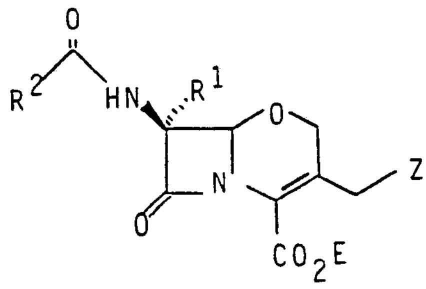

(4)

in der

$R^1$ die oben angegebene Bedeutung hat,

$R^2$-CO einen Acylrest,

Z für Chlor, Brom, Jod, Chloracetyl, Dichloracetyl, Trichloracetyl, O-Mesyl, O-Tosyl, O-Trifluormesyl oder (Methoxycarbonyl)acetyloxy steht;

E für eine in der β-Lactamchemie übliche Säureschutzgruppe darstellt,

die Säureschutzgruppe in an sich bekannter Weise abspaltet,

b) die so erhaltenen Verbindungen der Formel (3)

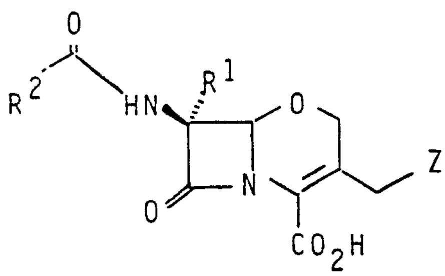

(3)

in der

$R^1$, $R^2$ und Z die obengenannten Bedeutungen haben, mit einem Pyridin der allgemeinen Formel

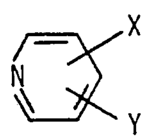

in der

X und Y die oben angegebenen Bedeutungen haben,

in einem geeigneten organischen Lösungsmittel zu Verbindungen der Formel (2)

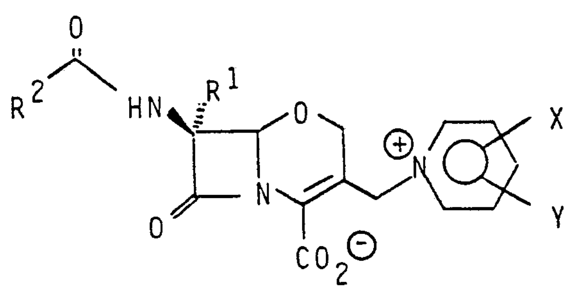

(2)

in welcher

$R^1$, $R^2$, X und Y die oben angegebenen Bedeutungen haben,

umsetzt und

c) den Acylrest $R^2$-CO in an sich bekannter Weise abspaltet.

5. Verfahren nach Anspruch 4, dadurch gekennzeichnet, daß die Abspaltung des Acylrestes im Verfahrensschritt c) enzymatisch erfolgt.

6. Verfahren nach den Ansprüchen 4 und 5, dadurch gekennzeichnet, daß $R^2$ für Phenyl, Phenylmethyl oder Phenoxymethyl steht.

7. Verfahren nach den Ansprüchen 4 - 6, dadurch gekennzeichnet, daß E für Diphenylmethyl oder tert.-Butyl steht.

8. Verwendung von Verbindungen nach Anspruch 1, 2 oder 3 zur Herstellung von pharmazeutisch aktiven β-Lactamverbindungen mit anderen Seitenketten als die Acylgruppe $R^2$-CO- in Formel (2).

**Revendications**

1. Composés de formule générale (1)

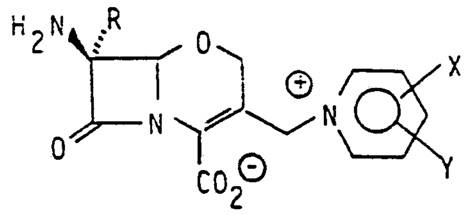

(1)

dans laquelle
R représente l'hydrogène ou un groupe méthoxy et
X et Y ont, indépendamment l'un de l'autre, les définitions suivantes:
hydrogène; halogène; cyano; hydroxy; alkoxy en $C_1$ à $C_4$; phénoxy; amino; alkylamino en $C_1$ à $C_4$, di(alkyle en $C_1$ à $C_4$) amino ou alcanoylamino en $C_1$ à $C_4$; carboxy; alcanoyle en $C_1$ à $C_4$; benzoyle; phényl-(alcanoyle en $C_1$ à $C_4$); (alkoxy en $C_1$ à $C_4$) carbonyle; sulfamoyle; carbamoyle; N-(alkyle en $C_1$ à $C_4$); N,N-dialkyle en $C_1$ à $C_4$ ou N-(alkoxy en $C_1$ à $C_4$)-carbonyl-carbamoyle; allophanoyle et 4(alkyle en $C_1$ à $C_4$)-allophanoyle; alkylsulfonyle en $C_1$ à $C_4$; alkylthio en $C_1$ à $C_4$; alkyle ou alcényle en $C_1$ à $C_6$, cycloalkyle ou cycloalcényle en $C_3$ à $C_7$ portant éventuellement un ou plusieurs substituants hydroxy, alkoxy en $C_1$ à $C_4$, halogéno, cyano, carboxy et (alkoxy en $C_1$ à $C_4$) carbonyle; alcanoyle en $C_1$ à $C_4$, (alkyle en $C_1$ à $C_4$) sulfonyle ou -sulfinyle, amino ou mono- ou di(alkyle en $C_1$ à $C_4$) amino; (alkoxy en $C_1$ à $C_4$) imino, un groupe phényle, hétérocycle pentagonal ou hexagonal avec 1 à 4 hétéroatomes du groupe O, N et/ou S, un groupe phénylalkyle ou hétérocycloalkyle ayant la définition indiquée ci-dessus avec 1 ou 2 atomes de carbone dans la partie alkyle, les restes hydrogène/hydrogène, amino/hydrogène et carbamoyle/hydrogène étant exclus;
X et Y forment conjointement un reste carbocyclique condensé dans les positions 2,3 et 4,5 au noyau de pyridine, avec 5 à 7 atomes de carbone qui sont éventuellement remplacés par des hétéroatomes O, S ou N.

2. Composés suivant la revendication 1, caractérisés en ce que X est un groupe 4-méthyle et Y est un groupe 2-méthyle ou 3-méthyle.

3. Composés suivant la revendication 1, caractérisés en ce que X est un groupe 4-hydroxy et Y est un groupe 2-méthyle ou bien X est un groupe 4-hydroxy et Y est un groupe 4-méthyle ou bien X est un groupe 3-méthyle et Y est un groupe 4-méthoxy ou bien X est un groupe 4-méthyle et Y est un groupe 3-méthoxy.

4. Procédé de préparation de composés de formule générale (1)

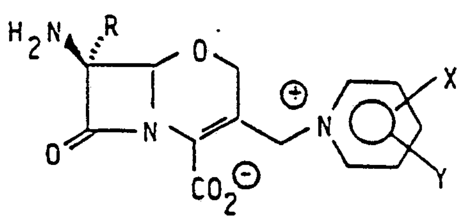

(1)

dans laquelle
R représente l'hydrogène ou un groupe méthoxy et
X et Y ont, indépendamment l'un de l'autre, les définitions suivantes:
hydrogène; halogène; cyano; hydroxy; alkoxy en $C_1$ à $C_4$; phénoxy; amino; alkylamino en $C_1$ à $C_4$, di(alkyle en $C_1$ à $C_4$) amino ou alcanoylamino en $C_1$ à $C_4$; carboxy; alcanoyle en $C_1$ à $C_4$; benzoyle; phényl-(alcanoyle en $C_1$ à $C_4$); (alkoxy en $C_1$ à $C_4$) carbonyle; sulfamoyle; carbamoyle; N-(alkyle en $C_1$ à $C_4$); N,N-dialkyle en $C_1$ à $C_4$ ou N-(alkoxy en $C_1$ à $C_4$) carbonyl-carbamoyle; allophanoyle et 4(alkyle en $C_1$ à $C_4$) allophanoyle; alkylsulfonyle en $C_1$ à $C_4$; alkylthio en $C_1$ à $C_4$; alkyle ou alcényle en $C_1$ à $C_6$, cycloalkyle ou cycloalcényle en $C_3$ à $C_7$ portant éventuellement un ou plusieurs substituants hydroxy, alkoxy en $C_1$ à $C_4$, halogéno, cyano, carboxy et (alkoxy en $C_1$ à $C_4$) carbonyle, alcanoyle en $C_1$ à $C_4$, (alkyle en $C_1$ à $C_4$) sulfonyle ou -sulfinyle, amino ou mono- ou di(alkyle en $C_1$ à $C_4$) amino, (alkoxy en $C_1$ à $C_4$) imino, un groupe phényle, hétérocycle pentagonal ou hexagonal avec 1 à 4 hétéroatomes du groupe O, N et/ou S, un groupe phénylalkyle ou hétérocycloalkyle ayant la définition indiquée ci-dessus avec 1 ou 2 atomes de carbone dans la partie alkyle;
X et Y forment conjointement un reste carbocyclique condensé dans les positions 2,3 et 4,5 au noyau de pyridine, avec 5 à 7 atomes de carbone qui sont éventuellement remplacés par des hétéroatomes O, S ou N,

caractérisé en ce que

(a) on scinde d'une manière connue le groupe protégeant la fonction acide dans les composés de formule générale (4)

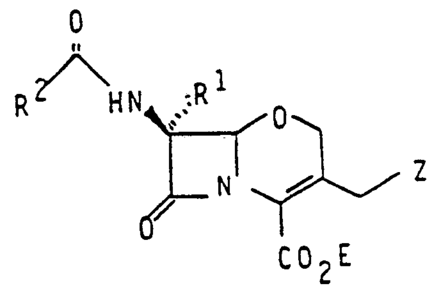

(4)

dans laquelle

$R^1$ a la définition indiquée ci-dessus,

$R^2$-CO représente un reste acyle,

Z représente le chlore, le brome, l'iode, un groupe chloracétyle, dichloracétyle, trichloracétyle, O-mésyle, O-tosyle, O-trifluorométhyle ou (méthoxycarbonyl) acétyloxy; E est un groupe protégeant la fonction acide d'emploi classique dans la chimie des β-lactames,

b) on fait réagir les composés ainsi obtenus de formule (3)

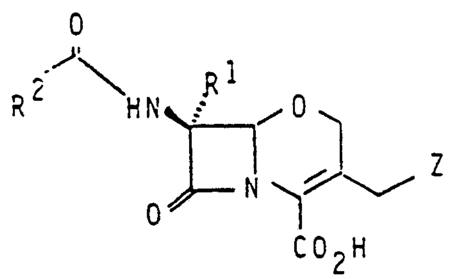

(3)

dans laquelle

$R^1$, $R^2$ et Z ont les définitions indiquées ci-dessus,

avec une pyridine de formule générale

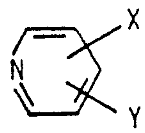

dans laquelle

X et Y ont les définitions indiquées ci-dessus, dans un solvant organique approprié pour former des composés de formule (2)

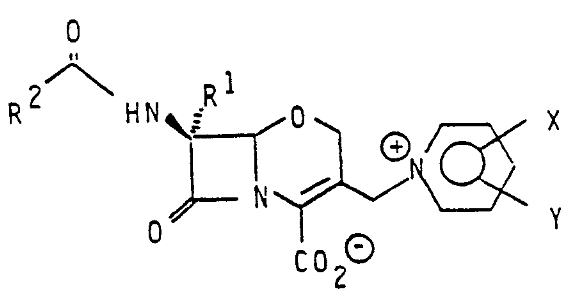

(2)

dans laquelle

$R^1$, $R^2$, X et Y ont les définitions indiquées ci-dessus, et

c) on scinde d'une manière connue le reste acyle $R^2$-CO.

5. Procédé suivant la revendication 4, caractérisé en ce que la scission du reste acyle est effectuée par voie enzymatique dans l'étape c) du procédé.

6. Procédé suivant les revendications 4 et 5, caractérisé en ce que $R^2$ représente un groupe phényle, phénylméthyle ou phénoxyméthyle.

7. Procédé suivant les revendications 4 à 6, caractérisé en ce que E représente le groupe diphénylméthyle ou tertio-butyle.

8. Utilisation de composés suivant les revendications 1, 2 ou 3 pour la préparation de composés du type β-lactame doués d'activité pharmaceutique, avec des chaînes latérales autres que le groupe acyle $R^2$-CO dans la formule (2).

## Claims

1. Compounds of the general formula (1)

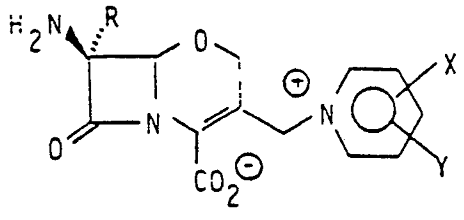

(1)

in which

R represents hydrogen or methoxy, and X and Y, independently of one another, represent the following meanings:

hydrogen; halogen; cyano; hydroxyl; $C_1$-$C_4$-alkoxy; phenoxy; amino, $C_1$-$C_4$-alkylamino, $C_1$-$C_4$-dialkylamino or $C_1$-$C_4$-alkanoylamino; carboxyl; $C_1$-$C_4$-alkanoyl; benzoyl; phenyl-$C_1$-$C_4$-alkanoyl; $C_1$-$C_4$-alkoxycarbonyl; sulphamoyl; carbamoyl; N-$C_1$-$C_4$-alkyl-, N,N-$C_1$-$C_4$-dialkyl- or N-$C_1$-$C_4$-alkoxycarbonylcarbamoyl; allophanoyl and 4-$C_1$-$C_4$-alkylallophanoyl; $C_1$-$C_4$-alkylsulphonyl; $C_1$-$C_4$-alkylthio; $C_1$-$C_6$-alkyl or alkenyl, $C_3$-$C_7$-cycloalkyl or cycloalkenyl, phenyl, heterocyclyl having 5- to 6-membered rings and 1 to 4 heteroatoms from the group comprising O, N and/or S, phenylalkyl or heterocyclylalkyl in the above mentioned meaning with 1 to 2 C atoms in the alkyl moiety, each of which is optionally substituted one or more times by hydroxyl, $C_1$-$C_4$-alkoxy, halogen, cyano, carboxyl and $C_1$-$C_4$-alkoxycarbonyl, $C_1$-$C_4$-alkanoyl, $C_1$-$C_4$-alkylsulphonyl or -sulphinyl, amino, or mono- or di-$C_1$-$C_4$-alkylamino, or or $C_1$-$C_4$-alkoxyimino, excepting the radicals hydrogen/hydrogen, amino/hydrogen and carbamoyl/hydrogen; or

X and Y together represent a carbocyclic radical which is fused to the pyridine ring in 2.3 or 4.5 position and has 5 - 7 carbon atoms which are optionally replaced by O, S or N heteroatoms.

2. Compounds according to claim 1, characterized in that X represents 4-methyl and Y represents 2-methyl or 3-methyl.

3. Compounds according to claim 1, characterized in that X represents 4-hydroxyl and Y represents 2-methyl or X represents 4-hydroxyl and Y represents 4-methyl or X represents 3-methyl and Y represents 4-methoxy or X represents 4-methyl and Y represents 3-methoxy.,

4. Process for the preparation of compounds of the general formula (1)

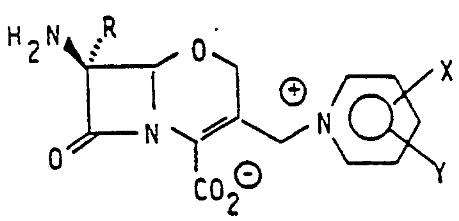

(1)

in which R represents hydrogen or methoxy, and X and Y, independently of one another, represent the following meanings:

hydrogen; halogen; cyano; hydroxyl; $C_1$-$C_4$-alkoxy; phenoxy; amino, $C_1$-$C_4$-alkylamino, $C_1$-$C_4$-dialkylamino or $C_1$-$C_4$-alkanoylamino; carboxyl; $C_1$-$C_4$-alkanoyl; benzoyl; phenyl-$C_1$-$C_4$-alkanoyl; $C_1$-$C_4$-alkoxycarbonyl; sulphamoyl; carbamoyl; N-$C_1$-$C_4$-alkyl-, N,N-$C_1$-$C_4$-dialkyl- or N-$C_1$-$C_4$-alkoxycarbonylcarbamoyl; allophanoyl and 4-$C_1$-$C_4$-alkylallophanoyl; $C_1$-$C_4$-alkylsulphonyl; $C_1$-$C_4$-alkylthio; $C_1$-$C_6$-alkyl or alkenyl, $C_3$-$C_7$-cycloalkyl or cycloalkenyl, phenyl, heterocyclyl having 5- to 6-membered rings and 1 to 4 heteroatoms from the group comprising O, N and/or S, phenylalkyl or heterocyclylalkyl in the above mentioned meaning with 1 to 2 C atoms in the alkyl moiety, each of which is optionally substituted one or more times by hydroxyl, $C_1$-$C_4$-alkoxy, halogen, cyano, carboxyl and $C_1$-$C_4$-alkoxycarbonyl, $C_1$-$C_4$-alkanoyl, $C_1$-$C_4$-alkylsulphonyl or -sulphinyl, amino, or mono- or di-$C_1$-$C_4$-alkylamino, or $C_1$-$C_4$-alkoxyimino; or

X and Y together represent a carbocyclic radical which is fused to the pyridine ring in 2.3 or 4.5 position and has 5 - 7 carbon atoms which are optionally replaced by O, S or N heteroatoms,

characterized in that

a) the acid-protecting group in the compounds of the general formula (4)

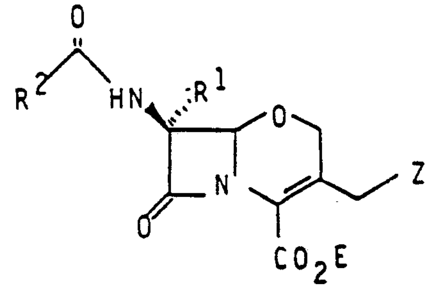

(4)

in which $R^1$ has the above mentioned meaning, $R^2$-CO is an acyl radical, Z represents chlorine, bromine, iodine, chloroacetyl, dichloroacetyl, trichloroacetyl, O-mesyl, O-tosyl, O-trifluoromesyl or (methoxycarbonyl)acetyloxy, and E represents and acid-protecting group customary in α-lactam chemistry, is eliminated in a manner known per se,

b) the compounds of the formula (3) obtained in this way

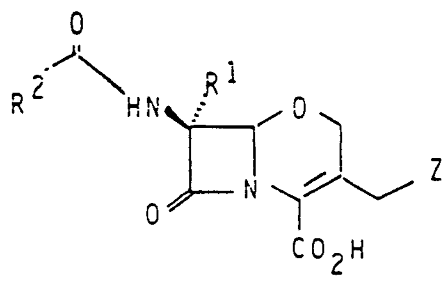

(3)

in which $R^1$, $R^2$ and Z have the above mentioned meanings, are reacted with a pyridine of the general formula

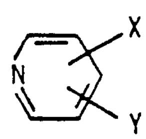

in which X and Y have the above mentioned meanings, in a suitable organic solvent to give compounds of the formula (2)

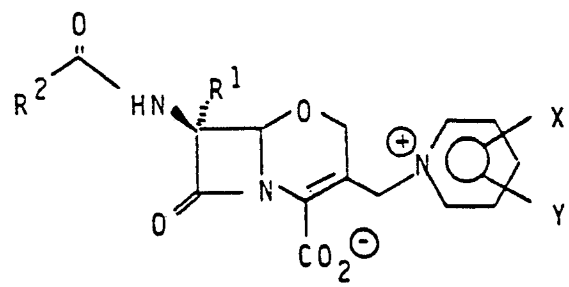

(2)

in which $R^1$, $R^2$, X and Y have the above mentioned meanings, and

c) the acyl radical $R^2$-CO is eliminated in a manner known per se.

5. Process according to claim 4, characterized in that the elimination of the acyl radical in process step c) is carried out enzymatically.

6. Process according to claims 4 and 5, characterized in that $R^2$ represents phenyl, phenylmethyl or phenoxymethyl.

7. Process according to claims 4 - 6, characterized in that E represents diphenylmethyl or tert.-butyl.

8. Use of compounds according to claim 1, 2 or 3 for the preparation of pharmaceutically active β-lactam compounds having side chains other than the acyl group $R^2$-CO- in formula (2).